# FASCICULE DE BREVET EUROPEEN

(11) **EP 3 384 291 B1**
(45) Date de publication et mention de la délivrance du brevet: **01.04.2020**
(21) Numéro de dépôt: 16819600.4
(22) Date de dépôt: 02.12.2016
(51) Int. Cl.: G01N 33/574, C12N 5/095

(54) **METHODE D'ISOLEMENT DE CELLULES SOUCHES CANCEREUSES**
VERFAHREN ZUR ISOLIERUNG VON KREBSSTAMMZELLEN
METHOD FOR ISOLATING CANCER STEM CELLS

(30) Priorité: 02.12.2015 FR 1561764
(43) Date de publication de la demande: 10.10.2018
(73) Titulaire: Université de Limoges, 87032 Limoges (FR)
(72) Inventeur: LACROIX, Aurélie, 87350 Panazol (FR); VARNAT, Frédéric, 87350 Panazol (FR); MICALLEF, Ludovic, 87220 Feytiat (FR); LALLOUE, Fabrice, 87170 Isle (FR); JAUBERTEAU, Marie-Odile, 87100 Limoges (FR)
(74) Mandataire: Grosset-Fournier, Chantal Catherine
(86) Numéro de dépôt international: PCT/FR2016/053197
(87) Numéro de publication internationale: WO 2017/093697

(56) Documents cités:
- WO-A2-2008/036419
- CAROL TUCKER-BURDEN ET AL: "Lectins Identify Glycan Biomarkers on Glioblastoma-Derived Cancer Stem Cells", STEM CELLS AND DEVELOPMENT, vol. 21, no. 13, 1 septembre 2012 (2012-09-01), pages 2374-2386, XP055157421, ISSN: 1547-3287, DOI: 10.1089/scd.2011.0369
- ARISTIDES G. VAIOPOULOS ET AL: "Colorectal Cancer Stem Cells", STEM CELLS., vol. 30, no. 3, 9 janvier 2012 (2012-01-09), pages 363-371, XP055295355, US ISSN: 1066-5099, DOI: 10.1002/stem.1031 cité dans la demande

## Description

La présente invention concerne le domaine de l'isolement des cellules souches cancéreuses.

Le cancer colorectal (CCR) est la troisième maladie la plus fréquente dans le monde. Celle-ci comme tout cancer, peut se résumer par une prolifération cellulaire anormale dans un tissu sain (ici la muqueuse colique) provoquant l'apparition d'une masse tumorale. L'une des hypothèses émises afin d'expliquer la progression tumorale ainsi que les mécanismes de résistance et les récidives repose sur l'existence de cellules souches cancéreuses. L'échappement thérapeutique de la tumeur aux traitements radio- et chimio-thérapeutique dépend de la présence de ces cellules au sein de la tumeur. Par conséquent la détection de ces cellules dans le tissu tumoral constitue un moyen de définir le niveau d'agressivité de la tumeur. La caractérisation de biomarqueurs spécifiques des cellules souches cancéreuses est d'un grand intérêt diagnostique et pronostique dans le traitement du cancer. Cependant, il n'existe pas actuellement de marqueurs spécifiques des cellules souches cancéreuses (CSCs) qui permettent leur discrimination avec certitude des autres cellules tumorales.

A cause de leur faible nombre et de l'absence de marqueurs spécifiques, les difficultés majeures pour étudier les CSCs résident dans leur isolement et leur caractérisation. La modification de protéines glycosylées est souvent associée à la progression tumorale et parait ainsi d'un intérêt majeur dans la recherche de marqueurs de surface.

Tucker-Burden et al. (Lectins identify glycan biomarkers on glioblastoma-derived cancer stem cells; Stem cells and development; 2012) décrit notamment l'utilisation de lectines reconnaissant le motif alpha-N-acetylgalactosamine pour la détection de cellules souches cancéreuses de glioblastome. Aristides et al. (Concise review : colorectal cancer stem cells; Stem cells; 2012) est une revue décrivant le rôle des cellules souches cancéreuses dans le cancer colorectal et les marqueurs utilisés, tels que CD133, CD44, ALDH-1, Msi-1 ou encore Lgr-5, pour la détection de cellules souches cancéreuses.

Il existe donc un besoin pour une méthode de détection et/ou d'isolement des cellules souches cancéreuses colorectales.

La présente invention vient combler ce besoin.

La présente invention repose sur la mise en évidence par les Inventeurs que le motif fucose α 1-2 galactose exprimé à la surface des cellules souches cancéreuses colorectales permet de détecter et d'isoler ces cellules en utilisant une lectine reconnaissant ce motif.

Dans un premier aspect, la présente invention concerne l'utilisation, en tant que moyen de marquage, d'une lectine, pour la détection et l'isolement des cellules souches cancéreuses colorectales.

Dans un second aspect, la présente invention concerne une méthode de détection et d'isolement de cellules souches cancéreuses colorectales comprenant le marquage des cellules souches cancéreuses colorectales avec une lectine. Avantageusement, la présente invention concerne l'utilisation d'une lectine et au moins un second moyen de marquage des cellules souches cancéreuses colorectales, notamment au moins une seconde lectine reconnaissant l'antigène T, en particulier deux lectines reconnaissant l'antigène T.

Dans un troisième aspect, la présente invention concerne un kit comprenant une lectine et un second moyen de détection et d'isolement des cellules cancéreuses colorectales.

Dans un quatrième aspect, la présente invention concerne une méthode de diagnostic de l'agressivité et/ou du risque de récidive d'un cancer colorectal pour définir une valeur pronostique pour l'adaptation thérapeutique d'un cancer colorectal, comprenant une étape de détection et d'isolement des cellules cancéreuses colorectales.

Un premier objet de la présente invention concerne l'utilisation, en tant que premier moyen de marquage, d'une lectine reconnaissant le motif fucose α 1-2 galactose, avantageusement *Ulex Europaeus Agglutinin 1* (UEA-1) ou *Trichosanthes Japonica Agglutinin II* (TJA-II), pour la mise en œuvre d'un procédé de détection et d'isolement des cellules souches cancéreuses colorectales.

La présente invention concerne l'utilisation, en tant que premier moyen de marquage, d'une lectine reconnaissant le motif fucose α 1-2 galactose, avantageusement *Ulex Europaeus Agglutinin 1* (UEA-1) ou *Trichosanthes Japonica Agglutinin II* (TJA-II), pour la mise en œuvre d'un procédé *in vitro* de détection et d'isolement des cellules souches cancéreuses colorectales, notamment dans un échantillon biologique colorectal.

La présente invention concerne également l'utilisation, en tant que premier moyen de marquage, d'une lectine reconnaissant le motif fucose α 1-2 galactose, avantageusement *Ulex Europaeus Agglutinin 1* (UEA-1) ou *Trichosanthes Japonica Agglutinin II* (TJA-II), pour la mise en œuvre d'un procédé *in vivo* de détection et d'isolement des cellules souches cancéreuses colorectales, notamment sur des modèles animaux ou sur des modèles humains de tumeurs.

Au sens de la présente invention, on entend par « moyen de marquage des cellules souches cancéreuses colorectales » une substance capable de se lier spécifiquement à un marqueur exprimé à la surface des cellules souches cancéreuses colorectales. Le moyen de marquage peut en particulier être un anticorps dirigé contre un déterminant antigénique, tel qu'une glycoprotéine, une protéine ou un glycane.

L'échantillon biologique colorectal peut notamment être une biopsie tumorale prélevée chez un patient atteint d'un cancer colorectal ou une biopsie prélevée chez un patient suspecté d'avoir un tel cancer.

L'échantillon biologique tumoral peut également être une lignée cellulaire cancéreuse colorectale ou une tumeur induite chez un animal par injection de lignées cellulaires cancéreuses, par exemple chez la souris ou le rat. La lignée cellulaire est de préférence une lignée cellulaire cancéreuse colorectale. Selon ce mode de réalisation, la tumeur induite contient des cellules souches cancéreuses colorectales qui sont avantageusement isolées des autres cellules de la tumeur afin d'être étudiées.

Au sens de la présente invention, on entend par « détection » le fait d'identifier par des méthodes spectroscopiques la présence de cellules souches cancéreuses colorectales au sein d'un tissu colorectal. Au sens de la présente invention, on entend par « isolement » le fait d'obtenir une population de cellules enrichie en cellules souches cancéreuses colorectales à partir d'une tumeur colorectale. Le terme « enrichi » désigne au sens de la présente invention une population de cellules dans laquelle le rapport nombre de cellules souches cancéreuses / nombre total de cellules est d'au moins 4, avantageusement d'au moins 6, de préférence d'au moins 8 et de manière particulièrement préférée au moins 9 tel que déterminé par le rapport cellules Epcam high + / cellules Epcam high - par cytométrie en flux.

Actuellement, le produit de référence pour l'isolement des cellules souches cancéreuses colorectales est constitué de billes magnétiques sur lesquelles sont greffés des anticorps anti-CD133 commercialisé par Miltenyi Biotec. Les Inventeurs de la présente invention ont mis en évidence qu'une lectine reconnaissant le motif fucose α 1-2 galactose était plus efficace pour isoler les cellules souches cancéreuses colorectales que ce système.

Il a également été mis en évidence par les Inventeurs que les cellules pouvaient être enrichies de manière particulièrement efficace en associant une lectine reconnaissant le motif fucose α 1-2 galactose et un second moyen de marquage des cellules souches cancéreuses colorectales.

La présente invention concerne donc également l'utilisation d'une lectine reconnaissant le motif fucose α 1-2 galactose et d'un second moyen de marquage des cellules souches cancéreuses colorectales pour la détection et l'isolement des cellules souches cancéreuses dans un échantillon biologique colorectal.

Le second moyen de marquage est au sens de la présente invention une substance reconnaissant de manière spécifique un ou plusieurs marqueurs exprimé à la surface des cellules souches cancéreuses. Il peut notamment s'agir d'un anticorps.

On peut citer, à titre d'exemple de marqueurs des cellules souches cancéreuses colorectales, CD133, CD44, CD166 (ALCAM), CD24, CD26, CD29, EpCAM, Oct-4 et Sox-2.

De préférence, le second moyen de marquage des cellules souches cancéreuses colorectales est une lectine reconnaissant l'antigène T ou un mélange de lectines reconnaissant l'antigène T.

De manière avantageuse, ladite lectine reconnaissant l'antigène T est choisie dans le groupe constitué de *Agaricus Bisporus Agglutinin* (ABA), *Amaranthus Caudatus Agglutinin* (ACA) et Jacaline.

Le second moyen de marquage des cellules souches cancéreuses colorectales peut également être un mélange de lectines reconnaissant l'antigène T, notamment choisi dans le groupe constitué de ABA et ACA ; ABA et Jacaline ; et Jacaline et ACA, avantageusement Jacaline et ACA.

Dans un mode de réalisation, la présente invention concerne l'utilisation d'un mélange de deux lectines choisi dans le groupe constitué de UEA-1 et ABA ; TJA-II et ABA ; UEA-1 et ACA ; TJA-II et ACA ; UEA-1 et Jacaline ; et TJA-II et Jacaline.

Dans un autre mode de réalisation, la présente invention concerne l'utilisation d'un mélange de trois lectines choisi dans le groupe constitué de UEA-1, ABA et ACA ; TJA-II, ABA et ACA ; UEA-1, ABA et Jacaline ; TJA-II, ABA et Jacaline ; UEA-1, Jacaline et ACA ; TJA-II, Jacaline et ACA, avantageusement UEA-1, ABA et Jacaline ; et UEA-1, Jacaline et ACA, de préférence UEA-1, Jacaline et ACA pour la mise en œuvre d'un procédé de détection et d'isolement des cellules souches cancéreuses colorectales dans un échantillon biologique colorectal.

Avantageusement, le mélange de trois lectines est constitué d'une lectine reconnaissant le motif fucose α 1-2 galactose : Jacaline : ABA ou ACA, dans un rapport molaire de 625 à 12500 : 16 à 320 : 1 à 20, avantageusement de 5000 à 7000 : 50 à 250 : 5 à 15, notamment de 6250 : 160 : 10.

Dans un mode de réalisation particulier, les lectines sont UEA-1, Jacaline et ACA, avantageusement dans un rapport molaire 625 à 12500 : 16 à 320 : 1 à 20, avantageusement de 5000 à 7000 : 50 à 250 : 5 à 15, de préférence de 6250 : 160 : 10.

Afin de détecter et isoler les cellules souches cancéreuses colorectales, la ou les lectines sont liées directement ou indirectement à un moyen de détection et d'isolement desdites cellules.

Par « moyen de détection et d'isolement », on entend au sens de la présente invention une substance ou un groupe de substances permettant d'identifier les moyens de marquage liés aux cellules souches cancéreuses colorectales. Ledit moyen de détection est par exemple un fluorophore, un chromophore, ou des billes magnétiques. Lorsque l'on souhaite isoler les cellules souches cancéreuses colorectales, on utilise de préférence un fluorophore ou des billes magnétiques.

Ledit moyen de détection et d'isolement peut être lié directement ou indirectement à la ou aux lectines.

Par « lié directement », on entend au sens de la présente invention que le moyen de détection et d'isolement est lié de manière covalente au moyen de marquage.

Par « lié indirectement », on entend au sens de la présente invention que le moyen de détection est lié à une seconde substance, la seconde substance étant capable de reconnaitre spécifiquement le moyen de marquage et de former avec lui une liaison suffisamment forte pour détecter et isoler les cellules souches cancéreuses colorectales. Le moyen de détection et d'isolement peut être notamment un système streptavidine / biotine.

Dans un mode de réalisation, le moyen de détection et d'isolement est constitué de biotine liée de manière covalente à la ou les lectines et de billes magnétiques sur lesquelles est greffée de la streptavidine, de l'avidine ou un anticorps anti-biotine.

La présente invention concerne donc l'utilisation d'une lectine biotinylée reconnaissant le motif fucose α 1-2 galactose, d'une première lectine biotinylée reconnaissant l'antigène T, d'une deuxième lectine biotinylée reconnaissant l'antigène T et de billes magnétiques sur lesquelles sont greffées de la streptavidine pour la mise en œuvre d'un procédé de détection et d'isolement des cellules souches cancéreuses colorectales. Avantageusement, lesdites lectines sont avantageusement UEA-1, Jacaline et ABA ou ACA, de préférence dans un rapport variant de 625 à 12500 : 16 à 320 : 1 à 20, avantageusement de 5000 à 7000 : 50 à 250 : 5 à 15, notamment de 6250 : 160 : 10.

Dans un autre mode de réalisation, le moyen de détection et d'isolement est un fluorophore. Le fluorophore peut être lié de manière covalente à la ou les lectines ou conjugué à de la streptavidine, à de l'avidine ou à un anticorps anti-biotine et la ou les lectines sont biotinylées.

Le fluorophore peut être tout fluorophore susceptible d'être utilisé pour la cytométrie en flux. De tels fluorophores sont disponibles dans le commerce. Il s'agit par exemple d'Alexa fluor 350, 405, 430, 488, 500, 514, 532, 546, 555, 568, 594, 610, 633, 647, 660, 680, 700, 750 ou 790, l'isothiocyanate de fluorescéine (FITC), la Rhodamine, l'allophycocyanine (APC) et la Phycoérythrine (PE). Avantageusement, le fluorophore est Alexa fluor 488, Alexa fluor 594 ou Alexa fluor 633.

La présente invention concerne donc l'utilisation d'une lectine biotinylée reconnaissant le motif fucose α 1-2 galactose, d'une première lectine biotinylée reconnaissant l'antigène T, d'une deuxième lectine biotinylée reconnaissant l'antigène T et d'un fluorophore conjugué à de l'avidine, de la streptavidine ou un anticorps anti-biotine pour la mise en œuvre d'un procédé de détection et d'isolement des cellules souches cancéreuses colorectales. Avantageusement, lesdites lectines sont UEA-1, Jacaline et ABA ou ACA, de préférence dans un rapport variant de 625 à 12500 : 16 à 320 : 1 à 20, avantageusement de 5000 à 7000 : 50 à 250 : 5 à 15, notamment de 6250 : 160 : 10.

La présente invention concerne donc également l'utilisation d'une lectine reconnaissant le motif fucose α 1-2 galactose, d'une première lectine reconnaissant l'antigène T, d'une deuxième lectine reconnaissant l'antigène T, lesdites lectines étant conjuguées à un fluorophore, pour la mise en œuvre d'un procédé de détection et d'isolement des cellules souches cancéreuses colorectales. Avantageusement, lesdites lectines sont UEA-1, Jacaline et ABA ou ACA, de préférence dans un rapport variant de 625 à 12500 : 16 à 320 : 1 à 20, avantageusement de 5000 à 7000 : 50 à 250 : 5 à 15, notamment de 6250 : 160 : 10.

L'étude des cellules souches cancéreuses à des fins de recherche et de diagnostique représente aujourd'hui une nécessité, en particulier pour mettre en évidence de nouvelles substances capables d'agir contre ces cellules. L'étude de ces cellules est également particulièrement utile dans le cadre de la médecine personnalisée.

Avantageusement, la présente invention concerne donc l'utilisation telle que décrite ci-dessus, pour l'isolement des cellules souches cancéreuses colorectales.

La détection des cellules souches cancéreuses colorectales, en particulier leur quantification, permet d'évaluer le également d'évaluer les risques de progression d'une tumeur. La présente invention concerne donc également l'utilisation d'une lectine reconnaissant le motif fucose α 1-2 galactose et éventuellement d'au moins une lectine reconnaissant l'antigène T dans les conditions décrites ci-dessus, pour le diagnostic *in vitro* du risque de récidive et/ou de l'agressivité d'un cancer colorectal pour définir une valeur pronostique pour l'adaptation thérapeutique d'un cancer colorectal.

Un deuxième objet de la présente invention concerne un procédé *in vitro* de détection et d'isolement des cellules souches cancéreuses colorectales (I), comprenant l'étape de marquage des cellules souches cancéreuses colorectales d'un échantillon biologique colorectal avec une lectine reconnaissant le motif fucose α 1-2 galactose, avantageusement UEA-1 ou TJA-II.

Le procédé selon la présente invention comprend, en tant qu'étape principale, le marquage des cellules souches cancéreuses colorectales avec une lectine reconnaissant le motif fucose α 1-2 galactose ou le marquage des cellules souches cancéreuses colorectales avec une lectine reconnaissant le motif fucose α 1-2 galactose et au moins une lectine reconnaissant l'antigène T.

La présente invention concerne également un procédé *in vitro* de détection et d'isolement des cellules souches cancéreuses colorectales (la), comprenant les étapes de :
(a) marquage des cellules souches cancéreuses colorectales avec une lectine reconnaissant le motif fucose α 1-2 galactose, pour obtenir un échantillon biologique dans lequel les cellules souches cancéreuses colorectales sont marquées,
(b) mise en contact de l'échantillon biologique dans lequel les cellules souches cancéreuses sont marquées avec un moyen de détection et d'isolement des cellules,
(c) détection des cellules souches cancéreuses colorectales,
(d) isolement des cellules souches cancéreuses colorectales.

Dans un mode de réalisation, le procédé selon la présente invention est mis en œuvre pour isoler les cellules souches cancéreuses colorectales. Cette étape d'isolement permet en particulier d'étudier les cellules souches cancéreuses colorectales détectées dans un échantillon tumoral colorectal afin, par exemple, de découvrir de nouveaux traitements capables d'éliminer ces cellules souches cancéreuses fréquemment à l'origine de récidives et de métastases.

L'échantillon biologique colorectal peut en particulier être une biopsie tumorale obtenue préalablement sur un patient atteint d'un cancer colorectal. L'échantillon biologique peut également être une biopsie d'un tissu colorectal chez un patient suspecté d'être atteint d'un cancer colorectal. L'échantillon biologique tumoral peut également être une lignée cellulaire cancéreuse colorectale ou une tumeur induite chez un animal par injection de lignées cellulaires cancéreuses, par exemple chez la souris ou le rat, notamment une lignée cellulaire humaine.

Avant le marquage des cellules souches cancéreuses colorectales à l'étape (a), les cellules sont avantageusement dissociées les unes des autres. Cette dissociation des cellules peut être réalisée selon des procédures conventionnelles, par exemple en utilisant une ou plusieurs enzymes capables de séparer les cellules les unes des autres sans altérer les glycanes exprimés à la surface des cellules, en particulier le motif fucose α 1-2 galactose et l'antigène T. La dissociation des cellules peut par exemple être mise en œuvre avec le mélange Liberase® commercialisé par la société Roche Diagnostic.

La présente invention concerne donc un procédé (I), ou (Ia) comprenant une étape de dissociation des cellules les unes des autres avant l'étape (a) de marquage.

Dans un mode de réalisation, l'étape de marquage (a) est mise en œuvre avec une lectine reconnaissant le motif fucose α 1-2 galactose en tant que premier moyen de marquage et un second moyen de marquage des cellules souches cancéreuses colorectales, avantageusement une lectine reconnaissant l'antigène T ou un mélange de lectines reconnaissant l'antigène T. La ou les lectines reconnaissant l'antigène T sont avantageusement choisies dans le groupe constitué de ABA, Jacaline et ACA. Avantageusement, l'étape (a) de marquage est mise en œuvre avec un mélange de lectines reconnaissant l'antigène T, notamment choisi dans le groupe constitué de ABA et ACA ; ABA et Jacaline ; et Jacaline et ACA, avantageusement Jacaline et ACA.

La présente invention concerne donc un procédé (I), ou (Ia) tel que décrit ci-dessus, dans lequel l'étape de marquage (a) est mise en œuvre avec un mélange de lectines choisi dans le groupe constitué de UEA-1, ABA et ACA ; TJA-II, ABA et ACA ; UEA-1, ABA et Jacaline ; TJA-II, ABA et Jacaline ; UEA-1, Jacaline et ACA ; TJA-II, Jacaline et ACA, avantageusement UEA-1, ABA et Jacaline ; et UEA-1, Jacaline et ACA, de préférence UEA-1, Jacaline et ACA.

De manière avantageuse, l'étape de marquage (a) est mise en œuvre avec un mélange lectine reconnaissant le motif fucose α 1-2 galactose : Jacaline : ABA ou ACA dans un rapport molaire variant de 625 à 12500 : 16 à 320 : 1 à 20, avantageusement de 5000 à 7000 : 50 à 250 : 5 à 15, notamment de 6250 : 160 : 10. De préférence, le mélange de lectines est constitué de UEA-1 : Jacaline : ABA ou ACA, dans un rapport molaire variant de 625 à 12500 : 16 à 320 : 1 à 20, avantageusement de 5000 à 7000 : 50 à 250 : 5 à 15, notamment de 6250 : 160 : 10, en particulier avec un mélange UEA-1, Jacaline et ACA dans un rapport molaire 6250 : 160 : 10.

A l'étape (b), l'échantillon biologique est mis en contact avec un moyen de détection et d'isolement tel que défini ci-dessus, c'est-à-dire des billes magnétiques sur lesquelles est greffée de la streptavidine, de l'avidine ou un anticorps anti-biotine, la ou les lectines étant biotinylées, d'un fluorophore lié de manière covalente à la lectine, ou d'un fluorophore conjugué à de la streptavidine, à de l'avidine ou à un anticorps anti-biotine, la ou les lectines étant biotinylées, le fluorophore étant tel que défini ci-dessus.

Les étapes (c) de détection et (d) d'isolement des cellules sont avantageusement mises en œuvre avec des techniques conventionnelles de détection et d'isolement des cellules telles que la cytométrie en flux ou le tri cellulaire magnétique.

Dans le procédé tel que décrit ci-dessus dans lequel l'étape (a) est mise en œuvre avec un mélange de lectines, les cellules souches sont avantageusement marquées simultanément par la lectine reconnaissant le motif fucose α 1-2 galactose et la ou les lectines reconnaissant l'antigène T.

Dans un autre mode de réalisation, le marquage des cellules souches cancéreuses colorectales dans l'échantillon biologique avec la lectine reconnaissant le motif fucose α 1-2 galactose et la lectine reconnaissant l'antigène T ou le mélange de lectines reconnaissant l'antigène T est mis en œuvre de manière séquentielle.

Dans ce mode de réalisation, le procédé selon la présente invention comprend donc les étapes de :
(a) marquage des cellules souches cancéreuses colorectales avec une lectine reconnaissant le motif fucose α 1-2 galactose, pour obtenir un échantillon dans lequel les cellules souches cancéreuses colorectales sont marquées par la lectine reconnaissant le motif fucose α 1-2 galactose,
(b) détection et isolement des cellules marquées par la lectine reconnaissant le motif fucose α 1-2 galactose,
(c) marquage des cellules souches cancéreuses colorectales isolées avec un second moyen de marquage, de préférence une lectine reconnaissant l'antigène T ou un mélange de lectines reconnaissant l'antigène T,
(d) détection des cellules souches cancéreuses marquées par la lectine ou un mélange de lectines reconnaissant l'antigène T,
(e) isolement des cellules souches cancéreuses colorectales.

Dans cet autre mode de réalisation, l'étape d'isolement des cellules souches cancéreuses marquées par la lectine reconnaissant le motif fucose α 1-2 galactose (b) peut être suivie d'une étape d'amplification cellulaire. Ainsi, après l'isolement des cellules, celles-ci peuvent être mises en culture dans un milieu permettant d'augmenter la quantité de cellules souches cancéreuses colorectales avant d'être soumises à un marquage avec un second moyen de marquage, avantageusement une lectine reconnaissant l'antigène T ou un mélange de lectines reconnaissant l'antigène T. L'étape (c) peut, elle-aussi, être suivie d'une étape d'amplification cellulaire avant les étapes (d) et (e) de détection et d'isolement des cellules souches cancéreuses colorectales.

Lorsque le second moyen de marquage est un mélange de lectines, l'étape (c) peut être mise en œuvre avec une première lectine reconnaissant l'antigène T, les cellules marquées isolées peuvent être éventuellement soumises à une étape d'amplification cellulaire, puis les cellules isolées peuvent être marquées avec une seconde lectine reconnaissant l'antigène T.

Les Inventeurs ont mis en évidence qu'une lectine reconnaissant le motif fucose α 1-2 galactose, notamment UEA-1, éventuellement en présence d'une lectine reconnaissant l'antigène T ou un mélange de lectines reconnaissant l'antigène T, permet de détecter de manière particulièrement efficace les cellules souches cancéreuses colorectales mais aussi de les isoler.

La présente invention est particulièrement adaptée pour l'étude des cellules souches cancéreuses colorectales à des fins de recherche, par exemple pour l'étude de ces cellules dans une lignée cellulaire cancéreuse colorectale ou une tumeur induite chez un animal par injection de lignées cellulaires cancéreuses, notamment humaines, par exemple chez la souris ou le rat.

La présente invention est également particulièrement adaptée pour une application diagnostique chez les patients atteints d'un cancer colorectal, notamment dans le suivi de la maladie et/ou du traitement.

Les cellules souches cancéreuses colorectales sont en effet une population de cellules particulières qui, en raison de leur résistance aux traitements de chimiothérapie, conduisent à la reformation de la tumeur et à la récidive tumorale. La présente invention permet donc, en permettant la détection des cellules souches cancéreuses colorectales, d'évaluer le risque de récidive du cancer colorectal.

La détection et la quantification des cellules souches cancéreuses colorectales dans un tissu tumoral permettent également de déterminer l'agressivité du cancer et sa capacité à se développer.

La détection et la quantification des cellules souches cancéreuses colorectales s'inscrit également dans une démarche de médecine personnalisée. La détection des cellules souches cancéreuses colorectales dans l'échantillon biologique permet notamment d'évaluer la valeur pronostique du traitement et si nécessaire, d'adapter le traitement.

Un quatrième objet de la présente invention concerne donc également une méthode de diagnostic *in vitro* du risque de récidive du cancer colorectal et/ou de l'agressivité d'un cancer colorectal pour définir une valeur pronostique pour l'adaptation thérapeutique d'un cancer colorectal, comprenant l'étape de marquage des cellules souches cancéreuses colorectales d'un échantillon biologique colorectal avec une lectine reconnaissant le motif fucose α 1-2 galactose, avantageusement UEA-1 ou TJA-II, ou d'un kit comprenant une lectine reconnaissant le motif fucose α 1-2 galactose tel que défini précédemment.

La présente invention concerne plus particulièrement une méthode de diagnostic *in vitro* du risque de récidive du cancer colorectal et/ou de l'agressivité d'un cancer colorectal pour définir une valeur pronostique pour l'adaptation thérapeutique d'un cancer colorectal comprenant les étapes de :
(a) marquage des cellules souches cancéreuses colorectales avec une lectine reconnaissant le motif fucose α 1-2 galactose, en tant que premier moyen de marquage, pour obtenir un échantillon biologique dans lequel les cellules souches cancéreuses sont marquées,
(b) mise en contact de l'échantillon biologique dans lequel les cellules souches cancéreuses sont marquées avec un moyen de détection et d'isolement des cellules,
(c) détection et quantification des cellules souches cancéreuses colorectales,
(d) déduction du risque de récidive du cancer colorectal et/ou de l'agressivité du cancer colorectal pour définir une valeur pronostique pour l'adaptation thérapeutique d'un cancer colorectal à partir de la présence et de la quantité de cellules souches cancéreuses colorectales.

L'étape (a) peut être mise en œuvre avec une lectine reconnaissant le motif fucose α 1-2 galactose seule ou en présence d'un second moyen de marquage des cellules souches cancéreuses colorectales tel que défini ci-dessus, avantageusement une lectine reconnaissant l'antigène T, notamment choisie dans le groupe constitué de ABA, Jacaline et ACA ou un mélange de lectines reconnaissant l'antigène T, notamment choisi dans le groupe constitué de ABA et ACA ; ABA et Jacaline ; et Jacaline et ACA, avantageusement Jacaline et ACA.

La présente invention concerne en particulier une méthode de diagnostic *in vitro* du risque de récidive du cancer colorectal et/ou de l'agressivité d'un cancer colorectal pour définir une valeur pronostique pour l'adaptation thérapeutique d'un cancer colorectal, dans laquelle l'étape de marquage des cellules souches cancéreuses est mise en œuvre avec un mélange de lectines choisi dans le groupe constitué de UEA-1, ABA et ACA ; TJA-II, ABA et ACA ; UEA-1, Jacaline et ABA ; TJA-II, Jacaline et ABA ; UEA-1, Jacaline et ACA ; TJA-II, Jacaline et ACA, avantageusement UEA-1, Jacaline et ABA ; et UEA-1, Jacaline et ACA, de préférence UEA-1, Jacaline et ACA. Avantageusement, le rapport molaire lectine reconnaissant le motif fucose α 1-2 galactose : Jacaline : ABA ou ACA varie de 625 à 12500 : 16 à 320 : 1 à 20, avantageusement 5000 à 7000 : 50 à 250 : 5 à 15, notamment dans un rapport molaire 6250 : 160 : 10.

De manière plus avantageuse, les lectines sont UEA-1 : Jacaline : ABA ou ACA, dans un rapport molaire de 625 à 12500 : 16 à 320 : 1 à 20, avantageusement 5000 à 7000 : 50 à 250 : 5 à 15, notamment dans un rapport molaire 6250 : 160 : 10.

Dans un mode de réalisation préféré, les lectines sont UEA-1, Jacaline et ACA dans un rapport molaire 6250 : 160 : 10.

Dans un mode de réalisation, la ou les lectines sont liées à un moyen de détection et d'isolement des cellules souches cancéreuses colorectales tel que défini ci-dessus.

La présente invention concerne en particulier une méthode de diagnostic *in vitro* du risque de récidive du cancer colorectal et/ou de l'agressivité d'un cancer colorectal pour définir une valeur pronostique pour l'adaptation thérapeutique d'un cancer colorectal dans laquelle le moyen de détection et d'isolement des cellules est choisi dans le groupe constitué de billes magnétiques sur lesquelles est greffée de la streptavidine, de l'avidine ou un anticorps anti-biotine, la ou les lectines étant biotinylées ; un fluorophore lié de manière covalente à la ou les lectines ; ou un fluorophore conjugué à de la streptavidine, de l'avidine ou un anticorps anti-biotine, la ou les lectines étant biotinylées.

La présente méthode de diagnostic peut notamment être mise en œuvre en utilisant pour l'étape de détection (c) et d'isolement des cellules (d) la cytométrie en flux, le tri cellulaire magnétique ou l'immunohistochimie. Lorsque la méthode de détection et d'isolement des cellules est la cytométrie en flux, le fluorophore conjugué à de la streptavidine, de l'avidine ou un anticorps anti-biotine est avantageusement Alexa Fluor 488 ou Alexa Fluor 633. Lorsque la méthode de détection et d'isolement des cellules est l'immunohistochimie, le fluorophore conjugué à de la streptavidine, de l'avidine ou un anticorps anti-biotine est avantageusement Alexa Fluor 488 ou Alexa Fluor 594.

Un quatrième objet de la présente invention concerne un kit, notamment destiné à la détection et l'isolement des cellules souches cancéreuses colorectales, comprenant en tant que premier moyen de marquage une lectine reconnaissant le motif fucose α 1-2 galactose, avantageusement UEA-1 ou TJA-II, et un second moyen de marquage des cellules souches cancéreuses colorectales.

Avantageusement, le second moyen de marquage des cellules souches cancéreuses colorectales est une lectine reconnaissant l'antigène T ou un mélange de lectines reconnaissant l'antigène T, notamment choisie dans le groupe constitué de ABA, Jacaline et ACA.

Dans un mode de réalisation, le kit selon l'invention comprend, en tant que second moyen de marquage, un mélange de lectines reconnaissant l'antigène T, choisi dans le groupe constitué de ABA et ACA ; ABA et Jacaline ; et Jacaline et ACA, avantageusement Jacaline et ACA.

La présente invention concerne donc avantageusement un kit comprenant des lectines choisi parmi UEA-1, ABA et ACA ; TJA-II, ABA et ACA ; UEA-1, ABA et Jacaline ; TJA-II, ABA et Jacaline ; UEA-1, Jacaline et ACA ; TJA-II, Jacaline et ACA, avantageusement UEA-1, ABA et Jacaline ; et UEA-1, Jacaline et ACA, de préférence UEA-1, Jacaline et ACA.

Les lectines du kit selon l'invention peuvent être dans des récipients indépendants pour être combinées au moment de leur utilisation, ou combinées dans un seul récipient prêt à l'emploi.

Lorsque les lectines sont combinées dans un seul récipient prêt à l'emploi, le mélange de lectines est avantageusement constitué d'une lectine reconnaissant le motif fucose α 1-2 galactose : Jacaline : ABA ou ACA, dans un rapport molaire de 625 à 12500 : 16 à 320 : 1 à 20, avantageusement 5000 à 700 : 50 à 250 : 5 à 15, notamment dans un rapport 6250 : 160 : 10.

Avantageusement, le mélange de lectines combinées dans un seul récipient prêt à l'emploi est avantageusement constitué d'UEA-1 : Jacaline : ABA ou ACA, dans un rapport molaire de 625 à 12500 : 16 à 320 : 1 à 20, avantageusement 5000 à 700 : 50 à 250 : 5 à 15, notamment dans un rapport molaire 6250 : 160 : 10.

Dans un mode de réalisation avantageux, la présente invention concerne un kit comprenant un mélange UEA-1 : Jacaline : ABA ou ACA, combinées dans un seul récipient prêt à l'emploi, dans un rapport molaire 6250 : 160 : 10.

Le kit selon la présente invention peut avantageusement contenir un moyen de détection et éventuellement d'isolement des cellules tel que défini ci-dessus. Le moyen de détection est donc en particulier constitué de billes magnétiques sur lesquelles est greffée de la streptavidine, de l'avidine ou un anticorps anti-biotine, la ou les lectines étant alors biotinylées ; d'un fluorophore lié de manière covalente à la lectine ; ou d'un fluorophore conjugué à de la streptavidine, à de l'avidine ou à un anticorps anti-biotine, la ou les lectines étant alors biotinylées. Avantageusement, le fluorophore est tout fluorophore susceptible d'être utilisé pour la cytométrie en flux, avantageusement Alexa Fluor 488 ou Alexa Fluor 633.

La présente invention concerne donc également un kit **A** comprenant :
- une lectine reconnaissant le motif fucose α 1-2 galactose,
- une première lectine reconnaissant l'antigène T,
- une deuxième lectine reconnaissant l'antigène T,
- un moyen de détection et d'isolement des cellules.
lesdites lectines étant avantageusement UEA-1, Jacaline et ABA ou ACA, dans un rapport molaire de 625 à 12500 : 16 à 320 : 1 à 20, avantageusement 5000 à 700 : 50 à 250 : 5 à 15, notamment dans un rapport molaire 6250 : 160 : 10.

La présente invention concerne dans un premier mode de réalisation particulier, un kit **A1** comprenant :
- une lectine biotinylée reconnaissant le motif fucose α 1-2 galactose,
- une première lectine biotinylée reconnaissant l'antigène T,
- une deuxième lectine biotinylée reconnaissant l'antigène T,
- un moyen de détection et d'isolement des cellules constitué de billes magnétiques sur lesquelles est greffée de la streptavidine, de l'avidine ou un anticorps anti-biotine,
lesdites lectines étant avantageusement UEA-1, Jacaline, ABA ou ACA dans un rapport molaire de 625 à 12500 : 16 à 320 : 1 à 20, avantageusement 5000 à 700 : 50 à 250 : 5 à 15, notamment dans un rapport molaire 6250 : 160 : 10.

Avantageusement, le kit est un kit **A1-1** comprenant :
- UEA-1 biotinylée,
- ABA biotinylée ou ACA biotinylée, avantageusement ACA biotinylée,
- Jacaline biotinylée,
- des billes magnétiques sur lesquelles est greffée de la streptavidine, de l'avidine ou un anticorps anti-biotine.

Lesdites lectines dans le kit A1-1 peuvent être combinées dans un seul récipient prêt à l'emploi, dans un rapport molaire UEA-1 biotinylée : Jacaline biotinylée : ABA biotinylée ou ACA biotinylée de 625 à 12500 : 16 à 320 : 1 à 20, avantageusement 5000 à 7000 : 50 à 250 : 5 à 15, notamment dans un rapport molaire 6250 : 160 : 10.

La présente invention concerne également, dans un second mode de réalisation particulier, un kit A2 comprenant :
- une lectine biotinylée reconnaissant le motif fucose α 1-2 galactose,
- une première lectine biotinylée reconnaissant l'antigène T,
- une deuxième lectine biotinylée reconnaissant l'antigène T,
- un moyen de détection et d'isolement des cellules constitué d'un fluorophore conjugué à de la streptavidine, à de l'avidine ou à un anticorps anti-biotine.

Le fluorophore peut être tout fluorophore susceptible d'être utilisé pour la cytométrie en flux. De tels fluorophores sont disponibles dans le commerce. Il s'agit par exemple d'Alexa fluor 350, 405, 430, 488, 500, 514, 532, 546, 555, 568, 594, 610, 633, 647, 660, 680, 700, 750 ou 790, l'isothiocyanate de fluorescéine (FITC), la Rhodamine, l'allophycocyanine (APC) et la Phycoérythrine (PE). Avantageusement, le fluorophore est Alexa fluor 488, Alexa fluor 594 ou Alexa fluor 633.

Avantageusement, le kit est un kit **A2-1** comprenant :
- UEA-1 biotinylée,
- Jacaline biotinylée,
- ABA biotinylée ou ACA biotinylée, avantageusement ACA biotinylée,
- un fluorophore conjugué à de la streptavidine, à de l'avidine ou à un anticorps anti-biotine,

Lesdites lectines peuvent être combinées dans un seul récipient prêt à l'emploi, dans un rapport molaire UEA-1 biotinylée : Jacaline biotinylée : ABA biotinylée ou ACA biotinylée de 625 à 12500 : 16 à 320 : 1 à 20, avantageusement 5000 à 7000 : 50 à 250 : 5 à 15, notamment dans un rapport molaire 6250 : 160 : 10.

Le kit peut également être un kit **A3,** comprenant :
- une lectine reconnaissant le motif fucose α 1-2 galactose, conjuguée à un fluorophore, notamment tel que défini ci-dessus,
- une première lectine reconnaissant l'antigène T, conjuguée à un fluorophore, notamment tel que défini ci-dessus,
- une deuxième lectine reconnaissant l'antigène T, conjuguée à un fluorophore, notamment tel que défini ci-dessus.

Avantageusement, le kit est un kit **A3-1** comprenant :
- UEA-1 conjuguée à un fluorophore, notamment tel que défini ci-dessus
- Jacaline conjuguée à un fluorophore, notamment tel que défini ci-dessus.
- ABA ou ACA conjuguée à un fluorophore, notamment tel que défini ci-dessus,

Lesdites lectines peuvent être combinées dans un seul récipient prêt à l'emploi, dans un rapport molaire UEA-1 conjuguée à un fluorophore : Jacaline conjuguée à un fluorophore : ABA conjuguée à un fluorophore ou ACA conjuguée à un fluorophore de 625 à 12500 : 16 à 320: 1 à 20, avantageusement 5000 à 7000 : 50 à 250 : 5 à 15, notamment dans un rapport molaire 6250 : 160 : 10. La présente invention concerne également l'utilisation d'un kit tel que défini ci-dessus, pour la mise en œuvre d'un procédé de détection et d'isolement des cellules souches cancéreuses colorectales.

La présente invention concerne également l'utilisation d'un kit tel que défini ci-dessus, pour le diagnostic *in vitro* du risque de récidive et/ou de l'agressivité d'un cancer colorectal pour définir une valeur pronostique pour l'adaptation thérapeutique d'un cancer colorectal.

Un cinqiuème objet de la présente invention concerne un procédé *in vitro* de détection et d'isolement des cellules souches cancéreuses colorectales (I), comprenant l'étape de marquage des cellules souches cancéreuses colorectales d'un échantillon biologique colorectal avec une lectine reconnaissant le motif fucose α 1-2 galactose modifiée avec moyen de détection et d'isolement, avantageusement UEA-1 ou TJA-II.

Le procédé selon la présente invention comprend, en tant qu'étape principale, le marquage des cellules souches cancéreuses colorectales avec une lectine reconnaissant le motif fucose α 1-2 galactose ou le marquage des cellules souches cancéreuses colorectales avec une lectine reconnaissant le motif fucose α 1-2 galactose et au moins une lectine reconnaissant l'antigène T.

La présente invention concerne également un procédé *in vitro* de détection et d'isolement des cellules souches cancéreuses colorectales (la), comprenant les étapes de :
(e) marquage des cellules souches cancéreuses colorectales avec une lectine reconnaissant le motif fucose α 1-2 galactose modifiée avec moyen de détection et d'isolement, pour obtenir un échantillon biologique dans lequel les cellules souches cancéreuses colorectales sont marquées,
(f) mise en contact de l'échantillon biologique dans lequel les cellules souches cancéreuses sont marquées avec un moyen de détection et d'isolement des cellules,
(g) détection des cellules souches cancéreuses colorectales,
(h) isolement des cellules souches cancéreuses colorectales.

Dans un mode de réalisation, le procédé selon la présente invention est mis en œuvre pour isoler les cellules souches cancéreuses colorectales. Cette étape d'isolement permet en particulier d'étudier les cellules souches cancéreuses colorectales détectées dans un échantillon tumoral colorectal afin, par exemple, de découvrir de nouveaux traitements capables d'éliminer ces cellules souches cancéreuses fréquemment à l'origine de récidives et de métastases.

L'échantillon biologique colorectal peut en particulier être une biopsie tumorale obtenue préalablement sur un patient atteint d'un cancer colorectal. L'échantillon biologique peut également être une biopsie d'un tissu colorectal chez un patient suspecté d'être atteint d'un cancer colorectal. L'échantillon biologique tumoral peut également être une lignée cellulaire cancéreuse colorectale ou une tumeur induite chez un animal par injection de lignées cellulaires cancéreuses, par exemple chez la souris ou le rat, notamment une lignée cellulaire humaine.

Avant le marquage des cellules souches cancéreuses colorectales à l'étape (a), les cellules sont avantageusement dissociées les unes des autres. Cette dissociation des cellules peut être réalisée selon des procédures conventionnelles, par exemple en utilisant une ou plusieurs enzymes capables de séparer les cellules les unes des autres sans altérer les glycanes exprimés à la surface des cellules, en particulier le motif fucose α 1-2 galactose et l'antigène T. La dissociation des cellules peut par exemple être mise en œuvre avec le mélange Liberase® commercialisé par la société Roche Diagnostic.

La présente invention concerne donc un procédé (I), ou (Ia) comprenant une étape de dissociation des cellules les unes des autres avant l'étape (a) de marquage.

Dans un mode de réalisation, l'étape de marquage (a) est mise en œuvre avec une lectine reconnaissant le motif fucose α 1-2 galactose modifiée avec moyen de détection et d'isolement en tant que premier moyen de marquage et un second moyen de marquage des cellules souches cancéreuses colorectales, avantageusement une lectine reconnaissant l'antigène T modifiée avec moyen de détection et d'isolement ou un mélange de lectines reconnaissant l'antigène T modifiées avec moyen de détection et d'isolement. La ou les lectines reconnaissant l'antigène T sont avantageusement choisies dans le groupe constitué de ABA, Jacaline et ACA. Avantageusement, l'étape (a) de marquage est mise en œuvre avec un mélange de lectines reconnaissant l'antigène T modifiées avec moyen de détection et d'isolement, notamment choisi dans le groupe constitué de ABA et ACA ; ABA et Jacaline ; et Jacaline et ACA, avantageusement Jacaline et ACA.

La présente invention concerne donc un procédé (I), ou (la) tel que décrit ci-dessus, dans lequel l'étape de marquage (a) est mise en œuvre avec un mélange de lectines choisi dans le groupe constitué de UEA-1, ABA et ACA ; TJA-II, ABA et ACA ; UEA-1, ABA et Jacaline ; TJA-II, ABA et Jacaline ; UEA-1, Jacaline et ACA ; TJA-II, Jacaline et ACA, avantageusement UEA-1, ABA et Jacaline ; et UEA-1, Jacaline et ACA, de préférence UEA-1, Jacaline et ACA.

De manière avantageuse, l'étape de marquage (a) est mise en œuvre avec un mélange lectine reconnaissant le motif fucose α 1-2 galactose : Jacaline : ABA ou ACA dans un rapport molaire variant de 625 à 12500 : 16 à 320 : 1 à 20, avantageusement de 5000 à 7000 : 50 à 250 : 5 à 15, notamment de 6250 : 160 : 10. De préférence, le mélange de lectines est constitué de UEA-1 : Jacaline : ABA ou ACA, dans un rapport molaire variant de 625 à 12500 : 16 à 320 : 1 à 20, avantageusement de 5000 à 7000 : 50 à 250 : 5 à 15, notamment de 6250 : 160 : 10, en particulier avec un mélange UEA-1, Jacaline et ACA dans un rapport molaire 6250 : 160 : 10.

A l'étape (b), l'échantillon biologique est mis en contact avec un moyen de détection et d'isolement tel que défini ci-dessus, c'est-à-dire des billes magnétiques sur lesquelles est greffée de la streptavidine, de l'avidine ou un anticorps anti-biotine, la ou les lectines étant biotinylées, d'un fluorophore lié de manière covalente à la lectine, ou d'un fluorophore conjugué à de la streptavidine, à de l'avidine ou à un anticorps anti-biotine, la ou les lectines étant biotinylées, le fluorophore étant tel que défini ci-dessus.

Les étapes (c) de détection et (d) d'isolement des cellules sont avantageusement mises en œuvre avec des techniques conventionnelles de détection et d'isolement des cellules telles que la cytométrie en flux ou le tri cellulaire magnétique.

Dans le procédé tel que décrit ci-dessus dans lequel l'étape (a) est mise en œuvre avec un mélange de lectines, les cellules souches sont avantageusement marquées simultanément par la lectine reconnaissant le motif fucose α 1-2 galactose et la ou les lectines reconnaissant l'antigène T.

Dans un autre mode de réalisation, le marquage des cellules souches cancéreuses colorectales dans l'échantillon biologique avec la lectine reconnaissant le motif fucose α 1-2 galactose et la lectine reconnaissant l'antigène T ou le mélange de lectines reconnaissant l'antigène T est mis en œuvre de manière séquentielle.

Dans ce mode de réalisation, le procédé selon la présente invention comprend donc les étapes de :
(f) marquage des cellules souches cancéreuses colorectales avec une lectine reconnaissant le motif fucose α 1-2 galactose modifiée avec moyen de détection et d'isolement, pour obtenir un échantillon dans lequel les cellules souches cancéreuses colorectales sont marquées par la lectine reconnaissant le motif fucose α 1-2 galactose,
(g) détection et isolement des cellules marquées par la lectine reconnaissant le motif fucose α 1-2 galactose,
(h) marquage des cellules souches cancéreuses colorectales isolées avec un second moyen de marquage, de préférence une lectine reconnaissant l'antigène T modifiée avec moyen de détection et d'isolement ou un mélange de lectines reconnaissant l'antigène T modifiées avec moyen de détection et d'isolement,
(i) détection des cellules souches cancéreuses marquées par la lectine ou un mélange de lectines reconnaissant l'antigène T,
(j) isolement des cellules souches cancéreuses colorectales.

Dans cet autre mode de réalisation, l'étape d'isolement des cellules souches cancéreuses marquées par la lectine reconnaissant le motif fucose α 1-2 galactose (b) peut être suivie d'une étape d'amplification cellulaire. Ainsi, après l'isolement des cellules, celles-ci peuvent être mises en culture dans un milieu permettant d'augmenter la quantité de cellules souches cancéreuses colorectales avant d'être soumises à un marquage avec un second moyen de marquage, avantageusement une lectine reconnaissant l'antigène T ou un mélange de lectines reconnaissant l'antigène T. L'étape (c) peut, elle-aussi, être suivie d'une étape d'amplification cellulaire avant les étapes (d) et (e) de détection et d'isolement des cellules souches cancéreuses colorectales.

Lorsque le second moyen de marquage est un mélange de lectines, l'étape (c) peut être mise en œuvre avec une première lectine reconnaissant l'antigène T, les cellules marquées isolées peuvent être éventuellement soumises à une étape d'amplification cellulaire, puis les cellules isolées peuvent être marquées avec une seconde lectine reconnaissant l'antigène T.

Les Inventeurs ont mis en évidence qu'une lectine reconnaissant le motif fucose α 1-2 galactose, notamment UEA-1, éventuellement en présence d'une lectine reconnaissant l'antigène T ou un mélange de lectines reconnaissant l'antigène T, permet de détecter de manière particulièrement efficace les cellules souches cancéreuses colorectales mais aussi de les isoler.

La présente invention est particulièrement adaptée pour l'étude des cellules souches cancéreuses colorectales à des fins de recherche, par exemple pour l'étude de ces cellules dans une lignée cellulaire cancéreuse colorectale ou une tumeur induite chez un animal par injection de lignées cellulaires cancéreuses, notamment humaines, par exemple chez la souris ou le rat.

La présente invention est également particulièrement adaptée pour une application diagnostique chez les patients atteints d'un cancer colorectal, notamment dans le suivi de la maladie et/ou du traitement. Les cellules souches cancéreuses colorectales sont en effet une population de cellules particulières qui, en raison de leur résistance aux traitements de chimiothérapie, conduisent à la reformation de la tumeur et à la récidive tumorale. La présente invention permet donc, en permettant la détection des cellules souches cancéreuses colorectales, d'évaluer le risque de récidive du cancer colorectal.

La détection et la quantification des cellules souches cancéreuses colorectales dans un tissu tumoral permettent également de déterminer l'agressivité du cancer et sa capacité à se développer.

La détection et la quantification des cellules souches cancéreuses colorectales s'inscrit également dans une démarche de médecine personnalisée. La détection des cellules souches cancéreuses colorectales dans l'échantillon biologique permet notamment d'évaluer la valeur pronostique du traitement et si nécessaire, d'adapter le traitement.

Un quatrième objet de la présente invention concerne donc également une méthode de diagnostic *in vitro* du risque de récidive du cancer colorectal et/ou de l'agressivité d'un cancer colorectal pour définir une valeur pronostique pour l'adaptation thérapeutique d'un cancer colorectal, comprenant l'étape de marquage des cellules souches cancéreuses colorectales d'un échantillon biologique colorectal avec une lectine reconnaissant le motif fucose α 1-2 galactose modifiée avec moyen de détection et d'isolement, avantageusement UEA-1 ou TJA-II, ou d'un kit comprenant une lectine reconnaissant le motif fucose α 1-2 galactose modifiée avec moyen de détection et d'isolement tel que défini précédemment.

La présente invention concerne plus particulièrement une méthode de diagnostic *in vitro* du risque de récidive du cancer colorectal et/ou de l'agressivité d'un cancer colorectal pour définir une valeur pronostique pour l'adaptation thérapeutique d'un cancer colorectal comprenant les étapes de :
(e) marquage des cellules souches cancéreuses colorectales avec une lectine reconnaissant le motif fucose α 1-2 galactose modifiée avec moyen de détection et d'isolement, en tant que premier moyen de marquage, pour obtenir un échantillon biologique dans lequel les cellules souches cancéreuses sont marquées,
(f) mise en contact de l'échantillon biologique dans lequel les cellules souches cancéreuses sont marquées avec un moyen de détection et d'isolement des cellules,
(g) détection et quantification des cellules souches cancéreuses colorectales,
(h) déduction du risque de récidive du cancer colorectal et/ou de l'agressivité du cancer colorectal pour définir une valeur pronostique pour l'adaptation thérapeutique d'un cancer colorectal à partir de la présence et de la quantité de cellules souches cancéreuses colorectales.

L'étape (a) peut être mise en œuvre avec une lectine reconnaissant le motif fucose α 1-2 galactose modifiée avec moyen de détection et d'isolement seule ou en présence d'un second moyen de marquage des cellules souches cancéreuses colorectales tel que défini ci-dessus, avantageusement une lectine reconnaissant l'antigène T modifiée avec moyen de détection et d'isolement, notamment choisie dans le groupe constitué de ABA, Jacaline et ACA ou un mélange de lectines reconnaissant l'antigène T, notamment choisi dans le groupe constitué de ABA et ACA ; ABA et Jacaline ; et Jacaline et ACA, avantageusement Jacaline et ACA.

La présente invention concerne en particulier une méthode de diagnostic *in vitro* du risque de récidive du cancer colorectal et/ou de l'agressivité d'un cancer colorectal pour définir une valeur pronostique pour l'adaptation thérapeutique d'un cancer colorectal, dans laquelle l'étape de marquage des cellules souches cancéreuses est mise en œuvre avec un mélange de lectines modifiées avec moyen de détection et d'isolement choisi dans le groupe constitué de UEA-1, ABA et ACA ; TJA-II, ABA et ACA ; UEA-1, Jacaline et ABA ; TJA-II, Jacaline et ABA ; UEA-1, Jacaline et ACA ; TJA-II, Jacaline et ACA, avantageusement UEA-1, Jacaline et ABA ; et UEA-1, Jacaline et ACA, de préférence UEA-1, Jacaline et ACA. Avantageusement, le rapport molaire lectine reconnaissant le motif fucose α 1-2 galactose : Jacaline : ABA ou ACA varie de 625 à 12500 : 16 à 320 : 1 à 20, avantageusement 5000 à 7000 : 50 à 250 : 5 à 15, notamment dans un rapport molaire 6250 : 160 : 10.

De manière plus avantageuse, les lectines sont UEA-1 : Jacaline : ABA ou ACA, dans un rapport molaire de 625 à 12500 : 16 à 320 : 1 à 20, avantageusement 5000 à 7000 : 50 à 250 : 5 à 15, notamment dans un rapport molaire 6250 : 160 : 10.

Dans un mode de réalisation préféré, les lectines sont UEA-1, Jacaline et ACA dans un rapport molaire 6250 : 160 : 10.

La présente invention concerne en particulier une méthode de diagnostic *in vitro* du risque de récidive du cancer colorectal et/ou de l'agressivité d'un cancer colorectal pour définir une valeur pronostique pour l'adaptation thérapeutique d'un cancer colorectal dans laquelle le moyen de détection et d'isolement des cellules est choisi dans le groupe constitué de billes magnétiques sur lesquelles est greffée de la streptavidine, de l'avidine ou un anticorps anti-biotine, la ou les lectines étant biotinylées ; un fluorophore lié de manière covalente à la ou les lectines ; ou un fluorophore conjugué à de la streptavidine, de l'avidine ou un anticorps anti-biotine, la ou les lectines étant biotinylées.

La présente méthode de diagnostic peut notamment être mise en œuvre en utilisant pour l'étape de détection (c) et d'isolement des cellules (d) la cytométrie en flux, le tri cellulaire magnétique ou l'immunohistochimie. Lorsque la méthode de détection et d'isolement des cellules est la cytométrie en flux, le fluorophore conjugué à de la streptavidine, de l'avidine ou un anticorps anti-biotine est avantageusement Alexa Fluor 488 ou Alexa Fluor 633. Lorsque la méthode de détection et d'isolement des cellules est l'immunohistochimie, le fluorophore conjugué à de la streptavidine, de l'avidine ou un anticorps anti-biotine est avantageusement Alexa Fluor 488 ou Alexa Fluor 594.

### DESCRIPTION DES FIGURES :

La **Figure 1** montre les résultats de la séparation de cellules souches cancéreuses sur un échantillon de cellules de la lignée HT29 avec des billes magnétiques sur lesquelles est greffé un anticorps anti-CD133 (T-AC133), avec le kit commercialisé par la société Miltenyi Biotec (CD133 MicroBead Kit) via le marqueur CD133 (prominin-1) glycosylé, avec des billes magnétiques sur lesquelles est greffée de la streptavidine et UEA-1 biotinylée (Lectine UEA-1), avec le mélange UEA-1 / Jacaline / ABA (Mix 1) et avec le mélange UEA-1 / Jacaline / ACA (Mix 2).

La **Figure 2** montre le ratio Epcam High + / Epcam high - après tri cellulaire avec UEA-1 seule, deux lectines, ou un mélange de trois lectines UEA-1 : Jacaline : ACA en proportions équimolaires (Mix 2) ou un mélange UEA-1 : Jacaline : ACA, dans un rapport molaire 6250 : 160 : 10.

La **Figure 3** montre les résultats du tri cellulaire avec des billes magnétiques sur différentes lignées de cellules cancéreuses colorectales avec le mélange UEA-1 / Jacaline / ABA (Mix 1S) et avec le mélange UEA-1 / Jacaline / ACA (Mix 2S).

La **Figure 4** montre les résultats d'un test de clonogénicité à partir de cellules non triées (T-), triées avec le kit Miltenyi Biotec AC133 (T-AC133), avec un mélange UEA-1 / Jacaline / ABA (Mix 1) ou un mélange UEA-1 / Jacaline / ACA (Mix2). L'axe des ordonnées est exprimé en pixel².

La **Figure 5** montre les résultats de la croissance tumorale *in vivo* après greffe de cellules non triées (T-, losanges), de cellules isolées avec UEA-1 / Jacaline / ACA (carrés) et de cellules de la fraction « négative » du tri (cellules cancéreuses non-souches, triangles) dans des souris *Nude.* Les résultats sont exprimés en volume (axe des ordonnées) en fonction du temps (en jours).

### EXEMPLES :

### Exemple 1 : Protocole d'isolement des cellules souches cancéreuses colorectales

### I. Matériels requis

### Réactifs et matériel

- Mix biotinylé marquant spécifiquement les Cellules Souches Cancéreuses du Cancer Colorectal (préparé à partir des lectines individuelles Vector Laboratories
- Kit CELLection Biotin Binder (*Invitrogen*)
- Aimant

### Tampons

- Versène (Invitrogen)
- Tampon 1 : PBS (Phosphate Buffer Saline sans Ca²⁺ et Mg²⁺) avec 0.1% BSA (Bovine Serum Albumine), pH 7.4
- Tampon 2 : PBS (Phosphate Buffer Saline sans Ca²⁺ et Mg²⁺) avec 0.1% BSA (Bovine Serum Albumine) et 0.6% de citrate de sodium
- Tampon 3 : RPMI 1640 avec 1% de FCS (Fetal Calf Serum), 1mM CaCl₂ et 5mM de MgCl₂, pH 7.0-7.4.

### II. Durée de l'expérimentation

- 20 min pour préparer les cellules
- 20 min pour marquer les cellules
- 20 min pour incuber les cellules marquées avec les billes
- 10 min pour récupérer la suspension non enrichie en CSCs
- 15 min pour casser la liaison CSCs/billes
- 5 min pour récupérer la suspension enrichie en CSCs d'intérêt

### - TOTAL : 1h30

### III. Mode opératoire par tri magnétique :

1. Préparation des cellules. Les cellules sont décollées de leur support avec du Versène pendant 10 min à 37°C.
2. Les cellules sont comptées et le nombre de cellules est ajusté à 1.10⁷.
3. La suspension cellulaire est centrifugée à 300g pendant 10 min puis le surnageant est éliminé.
4. Blocage des sites aspécifiques. 1mL de Tampon 2 est ajouté.
5. Marquage des cellules. 80µl de Mix est ajouté et le mélange incubé 10 min à 4°C.
6. 500µL de Tampon 2 est ajouté afin de laver les cellules et la suspension est centrifugée à 300g pendant 10 min et le surnageant est éliminé.
7. Billes. Les cellules sont resuspendues dans 1mL de Tampon 2 puis 25µL de billes préalablement lavées sont ajoutées et resuspendues à l'aide de Tampon 1. Le mélange est incubé 20 min à 4°C sous agitation douce.
8. Récupération de la suspension NON enrichie en CSCs. Le tube est alors placé sur l'aimant pendant 2 min puis le surnageant retiré, en le conservant dans un *falcon,* ceci alors que le tube est encore en place sur l'aimant.
9. Le tube est retiré de l'aimant, 1mL de Tampon 1 est ajouté, le tube est agité par vortex et replacé sur l'aimant pendant 2 min avant de conserver de nouveau le surnageant dans le même falcon qu'à l'étape 8. Cette étape est répétée deux fois.
10. Les cellules encore liées aux billes sont resuspendues à l'aide de 200µl de Tampon 3 préchauffé à 37°C. 4µL de tampon de coupure de la liaison cellules/billes constitué de DNaseI sont ajoutés. Ce mélange est incubé 15 min à température ambiante sous agitation douce.
11. La suspension est agitée avec une pipette vigoureusement 5 à 10 fois afin de faciliter la libération des cellules.
12. Récupération de la suspension enrichie en CSCs. Le tube est placé sur l'aimant pendant 2 min et le surnageant contenant les cellules d'intérêt transféré dans un tube contenant 200µL de tampon 3 préchauffé à 37°C. Les étapes 11 et 12 peuvent être répétées une nouvelle fois afin d'enrichir le rendement.

Ces expériences ont été réalisées dans des conditions similaires avec UEA-1 seul, un mélange de deux lectines ou avec le kit de la société Miltenyi et sur quatre autres lignées de cellules cancéreuses colorectales.

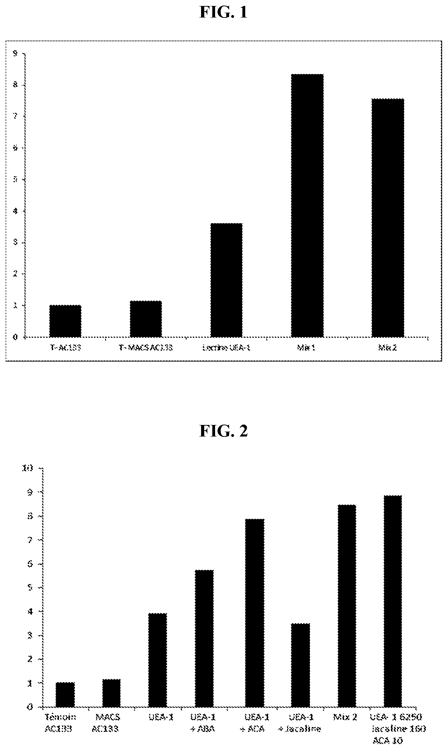

Les résultats de ces différents essais sont présentés dans les figures 1 à 3.

Ainsi que le montrent les résultats de ces essais, l'utilisation d'UEA-1 seule ou en mélange avec une ou deux lectines reconnaissant l'antigène T permet l'isolement des cellules souches cancéreuses colorectales avec une efficacité très nettement améliorée par rapport à la méthode standard (AC133).

### Exemple 2: Test de clonogénicité

Les tests de clonogénicité ont été réalisés dans une plaque à 6 puits à une densité de 500 cellules /cm² dans un milieu de composition MEM (Gibco) supplémenté de 50 unités/mL de pénicilline, 50 unités/mL de streptomycine (Gibco) et 2.4 g/L de bicarbonate de sodium, 1 M de tampon HEPES (Sigma Aldrich, Saint-Quentin-Fallavier, France), 1X progestérone (Sigma Aldrich), 1X putrescine (Sigma), 0.025 g/mL héparine (Sigma Aldrich), 30% (m/v) glucose (Sigma Aldrich,), 1X supplément de croissance B27 (Invitrogen, Carlsbad, CA), 20 ng/mL EGF (Sigma Aldrich), 20 ng/mL FGF humain basique (Sigma Aldrich), 1X supplément insuline-transferrine- sélénite de sodium (Roche diagnostics, Meylan, France).

L'évolution des colonosphères a été observée après incubation à 37 °C dans une atmosphère de CO₂ et quantifiée avec le logiciel ImageJ®.

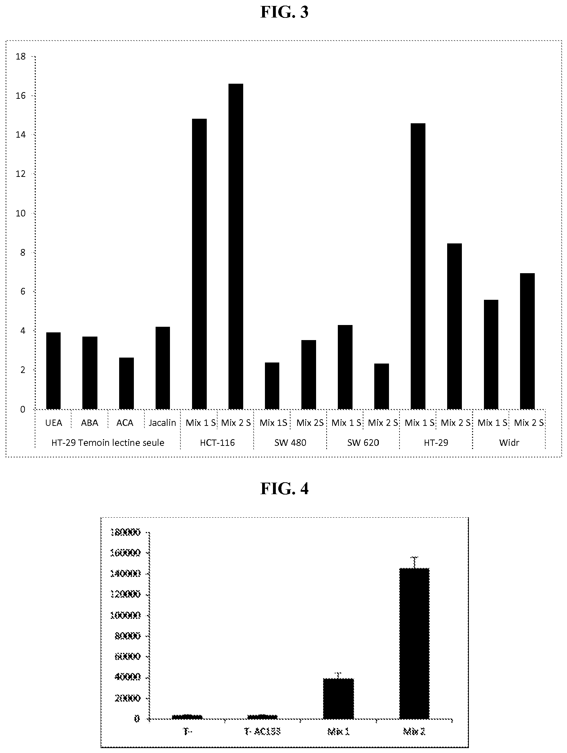

Les résultats sont présentés dans la figure 4.

Les cellules souches cancéreuses isolées selon la méthode décrite dans la présente invention conduisent à la formation de sphères de diamètre plus important que des cellules isolées avec un kit basé sur la reconnaissance du marqueur CD133.

La méthode selon la présente invention permet donc d'obtenir des cellules souches capables de reformer des tumeurs de manière nettement plus efficace que la méthode actuellement disponible et considérée comme le standard.

### Exemple 3 : Etude de l'évolution de tumeurs in vivo

Des essais *in vivo* adaptés de la méthode décrite dans Varnat F et al. EMBO Mol Med. 2009; 1 (6-7): 338-351 ont été réalisés.

Pour cela, 3x10⁶ cellules isolées avec le mélange UEA-1 / Jacaline / ACA ont été injectées en sous-cutané dans le flanc de souris *Nude* ou avec la fraction négative isolée avec le mélange.

Des souris contrôles ont été obtenues par injection de cellules n'ayant pas été triées au préalable (cellules issues de lignées HT-29 non triées).

Avant que les tumeurs n'aient atteint les limites légales, les animaux sont sacrifiés et les tumeurs étudiées.

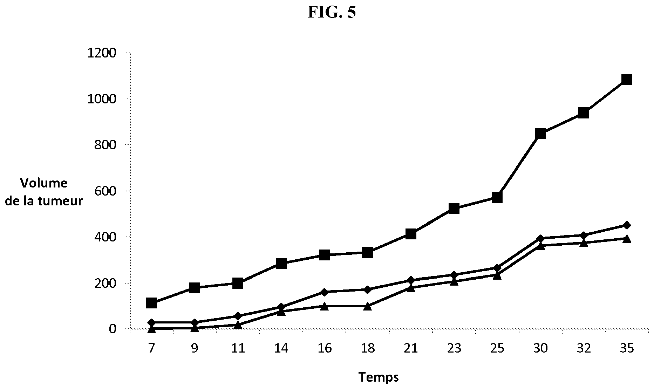

Les résultats de l'évolution de la dimension des tumeurs sont donnés dans la figure 5.

Comme dans l'expérience *in vitro* de l'exemple 4, les cellules souches cancéreuses colorectales conduisent à la formation de tumeurs de volume plus important que des cellules non-isolées. Les cellules isolées sont donc effectivement enrichies en cellules souches colorectales, initiatrices de tumeurs.

## Revendications

1. Utilisation, en tant que premier moyen de marquage, d'une lectine reconnaissant le motif fucose α 1-2 galactose, avantageusement UEA-1 ou TJA-II, pour la détection et l'isolement des cellules souches cancéreuses colorectales.

2. Utilisation selon la revendication 1, d'une lectine reconnaissant le motif fucose α 1-2 galactose et d'un second moyen de marquage des cellules souches cancéreuses colorectales, avantageusement choisi parmi une lectine reconnaissant l'antigène T, notamment ABA, Jacaline et ACA ou un mélange de lectines reconnaissant l'antigène T, notamment choisi dans le groupe constitué de ABA et ACA ; ABA et Jacaline ; et Jacaline et ACA, avantageusement Jacaline et ACA.

3. Utilisation selon l'une des revendications 1 à 2, dans laquelle la ou les lectines sont liées à un moyen de détection et d'isolement des cellules souches cancéreuses colorectales, avantageusement constitué de billes magnétiques sur lesquelles est greffée de la streptavidine, de l'avidine ou un anticorps anti-biotine, les lectines étant biotinylées ; d'un fluorophore lié de manière covalente à la lectine ; ou d'un fluorophore conjugué à de la streptavidine, à de l'avidine ou à un anticorps anti-biotine, les lectines étant biotinylées.

4. Utilisation selon l'une des revendications 1 à 3, dans laquelle :
• la lectine reconnaissant le motif fucose α 1-2 galactose est biotinylée,
• une première lectine reconnaissant l'antigène T est biotinylée,
• une deuxième lectine reconnaissant l'antigène T est biotinylée,
• le moyen de détection et d'isolement des cellules est constitué de billes magnétiques sur lesquelles sont greffées de la streptavidine.
lesdites lectines étant avantageusement UEA-1, Jacaline et ABA ou ACA, dans un rapport molaire de 625 à 12500 : 16 à 320 : 1 à 20, avantageusement 5000 à 7000 : 50 à 250 : 5 à 15, notamment dans un rapport molaire 6250 : 160 : 10.

5. Kit comprenant en tant que premier moyen de marquage une lectine reconnaissant le motif fucose α 1-2 galactose, avantageusement UEA-1 ou TJA-II, et une lectine reconnaissant l'antigène T, avantageusement choisie parmie ABA, ACA et Jacaline, ou un mélange de lectines reconnaissant l'antigène T, avantageusement choisi dans le groupe constitué de ABA et ACA ; ABA et Jacaline ; et Jacaline et ACA, avantageusement Jacaline et ACA,
lesdites lectines étant avantageusement UEA-1, Jacaline et ACA dans un rapport 6250 : 160 : 10.

6. Kit selon la revendication 5, comprenant en outre un moyen de détection et d'isolement des cellules, avantageusement constitué de billes magnétiques sur lesquelles est greffée de la streptavidine, de l'avidine ou un anticorps anti-biotine, les lectines étant biotinylées, d'un fluorophore lié de manière covalente à la lectine, ou d'un fluorophore conjugué à de la streptavidine, à de l'avidine ou à un anticorps anti-biotine, les lectines étant biotinylées.

7. Kit selon l'une des revendications 5 à 6, comprenant :
• une lectine biotinylée reconnaissant le motif fucose α 1-2 galactose,
• une première lectine biotinylée reconnaissant l'antigène T,
• une deuxième lectine biotinylée reconnaissant l'antigène T,
• un moyen de détection et d'isolement des cellules, avantageusement des billes magnétiques sur lesquelles est greffée de la streptavidine,
lesdites lectines étant avantageusement UEA-1, Jacaline, ABA ou ACA dans un rapport molaire de 625 à 12500 : 16 à 320 : 1 à 20, avantageusement 5000 à 7000 : 50 à 250 : 5 à 15, notamment dans un rapport molaire 6250 : 160 : 10 .

8. Procédé *in vitro* de détection et d'isolement des cellules souches cancéreuses colorectales, comprenant l'étape de marquage des cellules souches cancéreuses colorectales d'un échantillon biologique colorectal avec une lectine reconnaissant le motif fucose α 1-2 galactose, avantageusement UEA-1 ou TJA-II.

9. Procédé selon la revendication 8, comprenant les étapes de :
(a) marquage des cellules souches cancéreuses colorectales avec une lectine reconnaissant le motif **fucose α 1-2 galactose,** pour obtenir un échantillon biologique dans lequel les cellules souches cancéreuses colorectales sont marquées,
(b) mise en contact de l'échantillon biologique dans lequel les cellules souches cancéreuses sont marquées avec un moyen de détection et d'isolement des cellules,
(c) détection des cellules souches cancéreuses colorectales,
(d) éventuellement isolement des cellules souches cancéreuses colorectales.

10. Procédé selon l'une des revendications 8 ou 9, dans lequel l'étape (a) est mise en œuvre avec une lectine reconnaissant le motif fucose α 1-2 galactose en tant que premier moyen de marquage et un second moyen de marquage des cellules souches cancéreuses colorectales, avantageusement choisi dans le groupe constitué de ABA, Jacaline et ACA ou un mélange de lectines reconnaissant l'antigène T, notamment choisi dans le groupe constitué de ABA et ACA ; ABA et Jacaline ; et Jacaline et ACA, avantageusement Jacaline et ACA,
lesdites lectines étant avantageusement UEA-1, Jacaline et ACA dans un rapport molaire 6250 : 160 : 10.

11. Procédé selon la revendication 10, dans lequel la ou les lectines sont liées à un moyen de détection et d'isolement des cellules souches cancéreuses colorectales, avantageusement constitué de billes magnétiques sur lesquelles est greffée de la streptavidine, de l'avidine ou un anticorps anti-biotine, les lectines étant biotinylées, d'un fluorophore lié de manière covalente à la lectine, ou d'un fluorophore conjugué à de la streptavidine, à de l'avidine ou à un anticorps anti-biotine, les lectines étant biotinylées.

12. Méthode de diagnostic *in vitro* du risque de récidive du cancer colorectal et/ou de l'agressivité du cancer colorectal pour définir une valeur pronostique pour l'adaptation thérapeutique d'un cancer colorectal, comprenant une étape de marquage et d'isolement des cellules souches cancéreuses colorectales d'un échantillon biologique colorectal avec une lectine reconnaissant le motif fucose α 1-2 galactose, avantageusement UEA-1 ou TJA-II.

13. Méthode de diagnostic selon la revendication 12, comprenant les étapes de :
(a) marquage des cellules souches cancéreuses colorectales avec une lectine reconnaissant le motif fucose α 1-2 galactose, pour obtenir un échantillon biologique dans lequel les cellules souches cancéreuses sont marquées,
(b)mise en contact de l'échantillon biologique dans lequel les cellules souches cancéreuses sont marquées avec un moyen de détection et d'isolement des cellules,
(c) détection et quantification des cellules souches cancéreuses colorectales,
(d) déduction du risque de récidive du cancer colorectal et/ou de l'agressivité du cancer colorectal pour définir une valeur pronostique pour l'adaptation thérapeutique d'un cancer colorectal à partir de la présence et éventuellement de la quantité de cellules souches cancéreuses colorectales.

14. Méthode de diagnostic selon la revendication 12 ou 13, dans laquelle l'étape (a) est mise en œuvre avec une lectine reconnaissant le motif fucose α 1-2 galactose en tant que premier moyen de marquage et un second moyen de marquage des cellules souches cancéreuses colorectales, avantageusement choisi parmi une lectine reconnaissant l'antigène T notamment ABA, Jacaline ou ACA ou un mélange de lectines reconnaissant l'antigène T, notamment ABA et ACA ; ABA et Jacaline ; ou Jacaline et ACA, avantageusement Jacaline et ACA,
lesdites lectines étant avantageusement UEA-1, Jacaline et ACA dans un rapport molaire 6250 : 160 : 10.

15. Méthode de diagnostic selon l'une des revendications 12 à 14, dans laquelle le moyen de détection et d'isolement des cellules est constitué de billes magnétiques sur lesquelles est greffée de la streptavidine, de l'avidine ou un anticorps anti-biotine, les lectines étant biotinylées ; d'un fluorophore lié de manière covalente à la lectine ; ou d'un fluorophore conjugué à de la streptavidine, à de l'avidine ou à un anticorps anti-biotine, les lectines étant biotinylées.

## Patentansprüche

1. Verwendung eines Lektins, das die Fucose-α 1-2 Galactose-Einheit erkennt, vorteilhafterweise UEA-1 oder TJA-II, als erstes Markierungsmittel zur Erfassung und Isolierung der kolorektalen Krebsstammzellen.

2. Verwendung nach Anspruch 1 eines Lektins, das die Fucose-a 1-2 Galactose-Einheit erkennt, und eines zweiten Markierungsmittels der kolorektalen Krebsstammzellen, vorteilhafterweise ausgewählt unter einem Lektin, das das T-Antigen erkennt, insbesondere ABA, Jacalin und ACA oder ein Gemisch von Lektinen, die das T-Antigen erkennen, insbesondere ausgewählt in der Gruppe, die von ABA und ACA; ABA und Jacalin; und Jacalin und ACA, vorteilhafterweise Jacalin und ACA, gebildet ist.

3. Verwendung nach einem der Ansprüche 1 bis 2, bei der das oder die Lektine an ein Mittel zur Erfassung und Isolierung der kolorektalen Krebsstammzellen gebunden sind, vorteilhafterweise gebildet von Magnetkugeln, auf die Streptavidin, Avidin oder ein Anti-Biotin-Antikörper aufgepfropft ist, wobei die Lektine biotinyliert sind; von einem Fluorophor, das auf kovalente Weise an das Lektin gebunden ist; oder von einem Fluorophor, das an Streptavidin, an Avidin oder einen Anti-Biotin-Antikörper konjugiert ist, wobei die Lektine biotinyliert sind.

4. Verwendung nach einem der Ansprüche 1 bis 3, bei der:
• das Lektin, das die Fucose-a 1-2 Galactose-Einheit erkennt, biotinyliert ist,
• ein erstes Lektin, das das T-Antigen erkennt, biotinyliert ist,
• ein zweites Lektin, das das T-Antigen erkennt, biotinyliert ist,
• das Mittel zur Erfassung und Isolierung der Zellen von Magnetkugeln gebildet ist, auf die Streptavidin aufgepfropft ist,
wobei die Lektine vorteilhafterweise UEA-1, Jacalin und ABA oder ACA in einem Molverhältnis von 625 bis 12500 : 16 bis 320 : 1 bis 20, vorteilhafterweise 5000 bis 7000 : 50 bis 250 : 5 bis 15, insbesondere in einem Molverhältnis 6250 : 160 : 10 sind.

5. Set, umfassend als erstes Markierungsmittel ein Lektin, das die Fucose-a 1-2 Galactose-Einheit erkennt, vorteilhafterweise UEA-1 oder TJA-II, und ein Lektin, das das T-Antigen erkennt, vorteilhafterweise ausgewählt unter ABA, ACA und Jacalin, oder ein Gemisch von Lektinen, die das T-Antigen erkennen, vorteilhafterweise ausgewählt in der Gruppe, die von ABA und ACA; ABA und Jacalin; und Jacalin und ACA, vorteilhafterweise Jacalin und ACA, gebildet ist, wobei die Lektine vorteilhafterweise UEA-1, Jacalin und ACA in einem Verhältnis 6250 : 160 : 10 sind.

6. Set nach Anspruch 5, ferner umfassend ein Mittel zur Erfassung und Isolierung der Zellen, vorzugsweise gebildet von Magnetkugeln, auf die Streptavidin, Avidin oder ein Anti-Biotin-Antikörper aufgepfropft ist, wobei die Lektine biotinyliert sind, von einem Fluorophor, das an Streptavidin auf kovalente Weise gebunden ist, oder von einem Fluorophor, das an Streptavidin, Avidin oder einen Anti-Biotin-Antikörper konjugiert ist, wobei die Lektine biotinyliert sind.

7. Set nach einem der Ansprüche 5 bis 6, umfassend:
• ein Lektin, das die Fucose-a 1-2 Galactose-Einheit erkennt,
• ein erstes biotinyliertes Lektin, das das T-Antigen erkennt,
• ein zweites biotinyliertes Lektin, das das T-Antigen erkennt,
• ein Mittel zur Erfassung und Isolierung der Zellen, vorteilhafterweise Magnetkugeln, auf die Streptavidin aufgepfropft ist,
wobei die Lektine vorteilhafterweise UEA-1, Jacalin, ABA oder ACA in einem Molverhältnis von 625 bis 12500 : 16 bis 320 : 1 bis 20, vorteilhafterweise 5000 bis 7000 : 50 bis 250 : 5 bis 15, insbesondere in einem Molverhältnis 6250 : 160 :10 sind.

8. In-Vitro-Verfahren zur Erfassung und Isolierung der kolorektalen Krebsstammzellen, umfassend den Schritt des Markierens der kolorektalen Krebsstammzellen einer kolorektalen biologischen Probe mit einem Lektin, das die Fucose-a 1-2 Galactose-Einheit erkennt, vorteilhafterweise UEA-1 oder TJA-II.

9. Verfahren nach Anspruch 8, umfassend die folgenden Schritte:
(a) Markieren der kolorektalen Krebsstammzellen mit einem Lektin, das die **Fucose-α 1-2 Galactose-**Einheit erkennt, um eine biologische Probe zu erhalten, in der die kolorektalen Krebsstammzellen markiert sind,
(b) Inkontaktbringen der biologischen Probe, in der die Krebsstammzellen markiert sind, mit einem Mittel zur Erfassung und Isolierung der Zellen,
(c) Erfassung der kolorektalen Krebsstammzellen,
(d) eventuell Isolierung der kolorektalen Krebsstammzellen.

10. Verfahren nach einem der Ansprüche 8 oder 9, bei dem der Schritt (a) mit einem Lektin, das die Fucose-a 1-2 Galactose-Einheit erkennt, als erstem Markierungsmittel und einem zweiten Markierungsmittel der kolorektalen Krebsstammzellen eingesetzt wird, vorteilhafterweise ausgewählt in der Gruppe, die von ABA, Jacalin und ACA gebildet ist, oder einem Gemisch von Lektinen, die das T-Antigen erkennen, insbesondere ausgewählt in der Gruppe, die von ABA und ACA; ABA und Jacalin; und Jacalin und ACA, vorteilhafterweise Jacalin und ACA, gebildet ist,
wobei die Lektine vorteilhafterweise UEA-1, Jacalin und ACA in einem Molverhältnis 6250 : 160 : 10 sind.

11. Verfahren nach Anspruch 10, bei dem das oder die Lektine an ein Mittel zur Erfassung und Isolierung der kolorektalen Krebsstammzellen gebunden sind, das vorteilhafterweise von Magnetkugeln, auf die Streptavidin, Avidin oder ein Anti-Biotin-Antikörper aufgepfropft ist, wobei die Lektine biotinyliert sind, einem Fluorophor, das auf kovalente Weise an das Lektin gebunden ist, oder einem Fluorophor, das mit Streptavidin, Avidin oder einem Anti-Biotin-Antikörper konjugiert ist, gebildet ist, wobei die Lektine biotinyliert sind.

12. In-Vitro-Diagnoseverfahren der Rezidivgefahr des kolorektalen Krebses und/oder der Aggressivität des kolorektalen Krebses, um einen Prognosewert für die therapeutische Adaptation eines kolorektalen Krebses zu definieren, umfassend einen Schritt des Markierens und Isolierens der kolorektalen Krebsstammzellen einer kolorektalen biologischen Probe mit einem Lektin, das die Fucose-a 1-2 Galactose-Einheit erkennt, vorteilhafterweise UEA-1 oder TJA-II.

13. Diagnoseverfahren nach Anspruch 12, umfassend die folgenden Schritte:
(a) Markieren der kolorektalen Krebsstammzellen mit einem Lektin, das die Fucose-a 1-2 Galactose-Einheit erkennt, um eine biologische Probe zu erhalten, in der die kolorektalen Krebsstammzellen markiert sind,
(b) Inkontaktbringen der biologischen Probe, in der die Krebsstammzellen markiert sind, mit einem Mittel zur Erfassung und Isolierung der Zellen,
(c) Erfassung und Quantifizierung der kolorektalen Krebsstammzellen,
(d) Ableitung der Rezidivgefahr des kolorektalen Krebses und/oder der Aggressivität des kolorektalen Krebses, um einen Prognosewert für die therapeutische Adaptation eines kolorektalen Krebses aus der Präsenz und eventuell der Menge von kolorektalen Krebsstammzellen zu definieren.

14. Diagnoseverfahren nach Anspruch 12 oder 13, bei dem der Schritt (a) mit einem Lektin, das die Fucose-a 1-2 Galactose-Einheit erkennt, als erstem Markierungsmittel und einem zweiten Markierungsmittel der kolorektalen Krebsstammzellen eingesetzt wird, vorteilhafterweise ausgewählt unter einem Lektin, das das T-Antigen erkennt, insbesondere ABA, Jacalin oder ACA, oder einem Gemisch von Lektinen, die das T-Antigen erkennen, insbesondere ABA und ACA; ABA und Jacalin; oder Jacalin und ACA, vorteilhafterweise Jacalin und ACA,
wobei die Lektine vorteilhafterweise UEA-1, Jacalin und ACA in einem Molverhältnis 6250 : 160 : 10 sind.

15. Diagnoseverfahren nach einem der Ansprüche 12 bis 14, bei dem das Mittel zur Erfassung und Isolierung der kolorektalen Krebsstammzellen von Magnetkugeln, auf die Streptavidin, Avidin oder ein Anti-Biotin-Antikörper aufgepfropft ist, wobei die Lektine biotinyliert sind; einem Fluorophor, das auf kovalente Weise an das Lektin gebunden ist; oder einem Fluorophor, das mit Streptavidin, Avidin oder einem Anti-Biotin-Antikörper konjugiert ist, gebildet ist, wobei die Lektine biotinyliert sind.

## Claims

1. Use, as a first means of labelling, of a lectin recognizing the fucose α 1-2 galactose group, advantageously UEA-1 or TJA-II, for detecting and isolating colorectal cancer stem cells.

2. Use according to claim 1, of a lectin recognizing the fucose α 1-2 galactose group and a second means of labelling colorectal cancer stem cells, advantageously chosen from a lectin recognizing the T-antigen, in particular ABA, Jacaline and ACA or a mixture of lectins recognizing T-antigen, in particular chosen from the group consisting of ABA and ACA ; ABA and Jacaline ; and Jacaline and ACA, advantageously Jacaline and ACA.

3. Use according to one of claims 1 to 2, in which the lectin or lectins are bound to a means of detecting and isolating colorectal cancer stem cells, advantageously consisting of magnetic beads on which streptavidin, avidin or anti-biotin antibody is grafted, the lectins being biotinylated ; of a fluorophore covalently bound to the lectin ; or of a fluorophore conjugated with streptavidin, avidin or anti-biotin antibody, the lectins being biotinylated.

4. Use according to one of claims 1 to 3, in which:
• The lectin recognizing the fucose α 1-2 galactose group is biotinylated
• A first lectin recognizing the T-antigen is biotinylated
• A second lectin recognizing the T-antigen is biotinylated
• The means for detecting and isolating cells consists of magnetic beads on which streptavidin is grafted,
said lectins being advantageously UEA-1, Jacaline, ABA or ACA at a molar ratio of 625 to 12500: 16 to 320: 1 to 20, advantageously 5000 to 7000: 50 to 250: 5 to 15, in particular at a molar ratio of 6250: 160: 10.

5. Kit comprising as a first means of labelling a lectin recognizing the fucose α 1-2 galactose group, advantageously UEA-1 or TJA-II, and a lectin recognizing the T-antigen, advantageously chosen from ABA, ACA and Jacaline, or a mixture of lectins recognizing the T-antigen, advantageously chosen from the group consisting of ABA and ACA ; ABA and Jacaline ; and Jacaline and ACA, advantageously Jacaline and ACA,
said lectins being advantageously UEA-1, Jacaline and ACA at a ratio of 6250 : 160 : 10.

6. Kit according to claim 5, further comprising a means of detecting and isolating cells, advantageously consisting of magnetic beads on which streptavidin, avidin or anti-biotin antibody is grafted, the lectins being biotinylated, of a fluorophore covalently bound to the lectin, or of a fluorophore conjugated with streptavidin, avidin or anti-biotin antibody, the lectins being biotinylated.

7. Kit according to one of claims 5 to 6, comprising:
• a biotinylated lectin recognizing the fucose α 1-2 galactose group,
• A first biotinylated lectin recognizing the T-antigen,
• A second biotinylated lectin recognizing the T-antigen,
• The means for detecting and isolating cells, advantageously magnetic beads on which streptavidin is grafted,
said lectins being advantageously UEA-1, Jacaline, ABA or ACA at a molar ratio of 625 to 12500: 16 to 320: 1 to 20, advantageously 5000 to 7000: 50 to 250: 5 to 15, in particular at a molar ratio of 6250: 160: 10.

8. *In vitro* method for detecting and isolating colorectal cancer stem cells, comprising the step of labelling colorectal cancer stem cells from a colorectal biological sample with a lectin recognizing the fucose α 1-2 galactose group, advantageously UEA-1 or TJA-II.

9. Method according to claim 8, comprising steps of:
(a) labelling colorectal cancer stem cells with a lectin recognizing the fucose α 1-2 galactose group, to obtain a biological sample in which colorectal cancer stem cells are labeled,
(b) contacting the biological sample in which cancer stem cells are labelled with a means of detecting and isolating cells,
(c) detecting colorectal cancer stem cells,
(d) possibly, isolating colorectal cancer stem cells.

10. Method according to one of claims 8 or 9, in which step (a) is carried out with a lectin recognizing the fucose α 1-2 galactose group as the first means of labelling and a second means of labelling colorectal cancer stem cells, advantageously chosen from the group consisting of ABA, Jacaline and ACA or a mixture of lectins recognizing the T-antigen, in particular selected from the group consisting of ABA and ACA ; ABA and Jacaline ; and Jacaline and ACA, advantageously Jacaline and ACA,
said lectins being advantageously UEA-1, Jacaline and ACA at a molar ratio of 6250 : 160 : 10.

11. Method according to claim 10, in which the lectin or lectins are bound to a means of detecting and isolating colorectal cancer stem cells, advantageously consisting of magnetic beads on which streptavidin, avidin or anti-biotin antibody is grafted, the lectins being biotinylated, of a fluorophore covalently bound to the lectin, or of a fluorophore conjugated with streptavidin, avidin or an anti-biotin antibody, the lectins being biotinylated.

12. Method for the *in vitro* diagnosis of the risk of recurrence of colorectal cancer and/or aggressiveness of colorectal cancer to define a prognostic value for the therapeutic adaptation of a colorectal cancer, comprising a step of labelling and isolating colorectal cancer stem cells from a colorectal biological sample with a lectin recognizing the fucose α 1-2 galactose group, advantageously UEA-1 or TJA-II.

13. Method of diagnosis according to claim 12, comprising the steps of:
(a) labelling colorectal cancer stem cells with a lectin recognizing the fucose α 1-2 galactose group, to obtain a biological sample in which cancer stem cells are labeled,
(b) contacting the biological sample in which cancer stem cells are labeled with a means of detecting and isolating cells,
(c) detecting and quantifying colorectal cancer stem cells,
(d) deducing the risk of recurrence of colorectal cancer and/or aggressiveness of colorectal cancer to define a prognostic value for the therapeutic adaptation of a colorectal cancer from the presence and possibly the quantity of colorectal cancer stem cells.

14. Method of diagnosis according to claim 12 or 13, in which step (a) is carried out with a lectin recognizing the fucose α 1-2 galactose group, as a first means of labelling and a second means of labelling colorectal cancer stem cells, advantageously chosen from a lectin recognizing the T-antigen especially ABA, Jacaline or ACA or a mixture of lectins recognizing the T-antigen, especially ABA and ACA ; ABA and Jacaline ; or Jacaline and ACA, advantageously Jacaline and ACA,
said lectins being advantageously UEA-1, Jacaline and ACA at a molar ratio of 6250 : 160 : 10.

15. Method of diagnosis according to one of claims 12 to 14, in which the means of detecting and isolating cells consists of magnetic beads on which streptavidin, avidin or anti-biotin antibody is grafted, the lectins being biotinylated, of a fluorophore covalently bound to the lectin, or of a fluorophore conjugated with streptavidin, avidin or anti-biotin antibody, the lectins being biotinylated.
